(19) Europäisches Patentamt
European Patent Office
Office européen des brevets

(11) EP 2 536 438 B1

(12) **FASCICULE DE BREVET EUROPEEN**

(45) Date de publication et mention de la délivrance du brevet:
**04.12.2013 Bulletin 2013/49**

(21) Numéro de dépôt: **11705507.9**

(22) Date de dépôt: **18.02.2011**

(51) Int Cl.:
***A61K 51/04** (2006.01)* ***A61K 103/10** (2006.01)*

(86) Numéro de dépôt international:
**PCT/EP2011/052420**

(87) Numéro de publication internationale:
**WO 2011/101436 (25.08.2011 Gazette 2011/34)**

(54) **COMPOSITION POUR LE TRAITEMENT DU CANCER DU FOIE CHEZ L'HOMME A BASE DE RHENIUM-188 ET PROCEDE DE PREPARATION D'UNE TELLE COMPOSITION**

ZUSAMMENSETZUNG ZUR BEHANDLUNG VON LEBERKREBS BEI MENSCHEN AUF DER BASIS VON RHENIUM-188 UND VERFAHREN FÜR DIE ZUBEREITUNG EINER DERARTIGEN ZUSAMMENSETZUNG

COMPOSITION FOR TREATING LIVER CANCER IN HUMANS BASED ON RHENIUM-188 AND METHOD FOR PREPARING SUCH A COMPOSITION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.02.2010 FR 1000681**

(43) Date de publication de la demande:
**26.12.2012 Bulletin 2012/52**

(73) Titulaires:
- **Ecole Nationale Supérieure de Chimie de Rennes 35708 Rennes Cedex 7 (FR)**
- **Centre Eugène Marquis 35042 Rennes (FR)**
- **Université de Rennes I 35065 Rennes Cedex (FR)**

(72) Inventeurs:
- **NOIRET, Nicolas F-35250 St Sulpice La Foret (FR)**
- **GARIN, Etienne F-35520 La Meziere (FR)**
- **LEPAREUR, Nicolas F-35770 Vern Sur Seiche (FR)**
- **ARDISSON, Valérie F-35000 Rennes (FR)**

(74) Mandataire: **Larcher, Dominique
Cabinet Vidon,
16 B, rue Jouanet,
BP 90333
Technopole Atalante
35703 Rennes Cedex 7 (FR)**

(56) Documents cités:
**WO-A1-01/90114**

- **GARIN E ET AL: "Effect of a <188>Re-SSS lipiodol/<131>I-lipiodol mixture, <188>Re-SSS lipiodol alone or <131>I-lipiodol alone on the survival of rats with hepatocellular carcinoma", NUCLEAR MEDICINE COMMUNICATIONS 200604 GB LNKD- DOI: 10.1097/00006231-200604000-00008, vol. 27, no. 4, avril 2006 (2006-04), pages 363-369, XP008126387, ISSN: 0143-3636**
- **GARIN ETIENNE ET AL: "188Re-SSS lipiodol: radiolabelling and biodistribution following injection into the hepatic artery of rats bearing hepatoma", NUCLEAR MEDICINE COMMUNICATIONS, vol. 25, no. 10, octobre 2004 (2004-10), pages 1007-1013, XP008126388, ISSN: 0143-3636**
- **GARIN E ET AL: "Effect of Stabilized Iodized Oil Emulsion on Experimentally Induced Hepatocellular Carcinoma in Rats", JOURNAL OF VASCULAR AND INTERVENTIONAL RADIOLOGY, VA, vol. 16, no. 6, 1 juin 2005 (2005-06-01), pages 841-848, XP025345500, ISSN: 1051-0443 [extrait le 2005-06-01]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 536 438 B1

- **SANDRINE BALLOT ET AL: "99mTc/188Re-labelled lipid nanocapsules as promising radiotracers for imaging and therapy: formulation and biodistribution", EUROPEAN JOURNAL OF NUCLEAR MEDICINE AND MOLECULAR IMAGING, SPRINGER, BERLIN, DE LNKD- DOI: 10.1007/S00259-005-0007-0, vol. 33, no. 5, 1 février 2006 (2006-02-01), pages 602-607, XP019422341, ISSN: 1619-7089**
- **ANONYMOUS: "Tadeo. Automated Module for Synthesis of Theraupetic Radiopharmaceuticals", , [Online] pages 1-10, XP002600909, COMECER Extrait de l'Internet: URL:http://www.emnovation.nl/PDF/Radiochemistry_Target_SynthesisR.pdf> [extrait le 2010-09-16]**
- **JOURNAL D'INFORMATION DU CENTRE EUGÈNE MARQUIS, [Online] no. 20, juillet 2007 (2007-07), XP002600908, Extrait de l'Internet: URL:http://www.centre-eugene-marquis.fr/uploads/fichiers/journal%20CEM%2007-2007%20intranet.pdf> [extrait le 2010-09-16]**
- **OH S J ET AL: "Automated preparation of <188>Re-labeled radiopharmaceuticals for endovascular radiation therapy", APPLIED RADIATION AND ISOTOPES, ELSEVIER, OXFORD, GB LNKD- DOI:10.1016/S0969-8043(03)00170-2, vol. 59, no. 4, 1 octobre 2003 (2003-10-01), pages 225-230, XP004458714, ISSN: 0969-8043 cité dans la demande**
- **ANONYMOUS: "188RE-SSS Lipiodol to Treat HepatoCellular Carcinomas", , [Online] 18 mai 2010 (2010-05-18), XP002600910, Clinical Trials Feeds Extrait de l'Internet: URL:http://clinicaltrialsfeeds.org/clinica l-trials/show/NCT01126463> [extrait le 2010-09-17]**
- **LEPAREUR N ET AL: "Automation of labelling of Lipiodol with high-activity generator-produced <188>Re", APPLIED RADIATION AND ISOTOPES 2011 ELSEVIER LTD GBR LNKD- DOI: 10.1016/J.APRADISO.2010.11.001, vol. 69, no. 2, février 2011 (2011-02), pages 426-430, XP002630129, ISSN: 0969-8043**

## Description

### 1. Domaine de l'invention

Le domaine de l'invention est celui de la formulation de compositions chimiothérapeutiques pour le traitement du cancer du foie chez l'homme.

Plus précisément, la présente invention a pour objet une composition pour le traitement du cancer du foie chez l'homme comprenant un complexe de rhénium radioisotopique (III) à haute activité et une phase organique lipophile.

Elle met notamment en oeuvre un nouveau procédé pour le radiomarquage stable en phase lipophile par un complexe de rhénium radioisotopique (III) à haute activité, ainsi que l'automatisation de ce procédé.

### 2. Art antérieur

Le cancer du foie chez l'homme est une pathologie grave qui représente le cinquième cancer le plus commun dans le monde. Son incidence varie grandement selon les régions du monde. Il affecte plus particulièrement l'Asie du Sud-Est et l'Afrique, mais son incidence dans les pays développés, et particulièrement l'Europe, est en forte progression (E. Garin, P. Bourguet, in Ell and Gambir 3rd Ed., Nuclear Medicine in Clinical Diagnosis and Treatment, Hong-Kong: Churchill-Livingstone, 2004, 473-483).

Son pronostic est extrêmement faible. Les possibilités thérapeutiques sont limitées par le degré de sévérité de l'hépatopathie sous-jacente et par l'extension intra-hépatique de la maladie. La transplantation hépatique est le seul traitement réellement curatif traitant la tumeur et la maladie hépatique favorisante. La résection chirurgicale est également une méthode chirurgicale efficace. Elle n'est malheureusement applicable qu'à des patients non cirrhotiques, ce qui représente moins de 5 % des cas dans les pays occidentaux. Dans ce contexte, deux approches thérapeutiques semblent les plus prometteuses : la radiothérapie métabolique par voie intra-artérielle ou intra-tumorale et la chimioembolisation artérielle par le Lipiodol.

La radiothérapie métabolique est largement utilisée dans le traitement, curatif ou palliatif, de nombreux cancers, et notamment du cancer du foie. La radiothérapie métabolique des tumeurs hépatiques se fait principalement via l'artère hépatique. Le foie sain est irrigué en sang et oxygène à la fois par la veine porte et l'artère hépatique. Néanmoins, dans le cas des tumeurs, ces dernières, richement vascularisées, sont essentiellement irriguées par l'artère hépatique, tandis que l'irrigation des tissus sains se fait à 80 % par la veine porte.

La chimioembolisation artérielle a été développée pour améliorer les performances de la radiothérapie métabolique. Cette technique consiste à injecter dans l'artère hépatique du patient un mélange contenant une substance chimiothérapeutique associée à des particules qui provoquent une embolie, c'est-à-dire qu'elles bloquent le flux sanguin vers la tumeur.

Cette thérapie alternative à la résection chirurgicale et à la radiothérapie présente un double avantage :

- elle permet de libérer une dose très concentrée de substance chimiothérapeutique dans la tumeur en épargnant les tissus sains et le reste de l'organisme,
- l'embolie de la tumeur permet d'y bloquer le médicament, ce qui augmente son efficacité, tout en affaiblissant la tumeur par la privation de nutriments et d'oxygène.

De plus, les effets secondaires pour le patient sont grandement limités et cette thérapie semble donner un avantage en termes de survie aux patients traités par cette voie (B. Lambert, J.Nucl.Med., 2005, 46, 60-66).

Le vecteur le plus utilisé pour l'administration intra-hépatique est le Lipiodol. Le Lipiodol, également connu sous le nom d'Ethidiol® aux Etats Unis, est un agent de contraste liposoluble obtenu par iodation et estérification de l'huile d'oeillette (mélange d'acides linoléique, oléique, palmitique et stéarique), huile obtenue à base de graines de pavot. La proportion d'iode est d'environ 38 % en masse (soit 475 mg/mL). Le Lipiodol est sélectivement capté par les hépatocarcinomes (CHC) et par certaines métastases hépatiques, d'origine colonique, neuroendocrine et mammaire. Le Lipiodol a donc été utilisé pour la détection de l'hépatocarcinome et de ses éventuelles tumeurs satellites non détectables par les méthodes usuelles d'imagerie, puis également pour véhiculer des substances chimiothérapeutiques. Il a également été montré un temps de rétention intra-tumoral largement supérieur au temps de rétention dans le foie sain, avec une rétention dans l'hépatocarcinome pouvant atteindre plusieurs mois. La persistance du Lipiodol au sein des tumeurs a amené à la proposition d'un marquage covalent de celui-ci par de l'iode-131 radioactif ($^{131}I$) afin d'effectuer une radiothérapie ciblée, l'iode « froid », c'est-à-dire non radioactif, contenu dans le Lipiodol étant remplacé par de l'iode radioactif. Cependant, la durée de vie de l'iode-131 ($^{131}I$) n'est que de 8 jours et cet élément est la source d'importantes émissions de rayonnements $\gamma$. Par conséquent, l'intérêt pour le développer en thérapie clinique de routine a rapidement décliné.

Par ailleurs, les propriétés de l'iode-131 étant sub-optimales, d'autres radioéléments, avec des caractéristiques

plus adaptées, ont été proposés, notamment l'yttrium-90 ($^{90}Y$) et le rhénium-186 ou 188 ($^{186}Re$, $^{188}Re$). Toutefois, l'yttrium-90 est coûteux et difficile à imager, et des risques de relargage, conduisant à une irradiation médullaire indésirable, ne sont pas à négliger, ce qui limite son développement (S.J. Wang et al., J. Nucl. Med., 1996, 37, 332-335).

En ce qui concerne le rhénium, deux stratégies de marquage ont été décrites. La première, similaire au marquage à l'Iode-131, consiste en un marquage covalent du Lipiodol (S.J. Wang et al., Appl. Radiat. Isot., 1996, 47, 267-271), *via* un chélate EDTB (N,N,N',N'-tetrakis(2-benzymidazoylmethyl) 2-ethanediamine). Cette méthode consiste à mettre en présence le chélate EDTB dissous dans de l'acide acétique glacial en présence de Lipiodol. L'acide acétique est éliminé de ce mélange par barbotage d'Argon sur la nuit et le chélate se fixe à la chaîne aliphatique des acides gras composant le Lipiodol. Le Lipiodol ainsi marqué par le chélate EDTB est mis en présence avec le $^{188}Re$. Une réaction d'oxydoréduction se forme entre le chélate EDTB et le rhénium pour produire du Lipiodol radiomarqué.

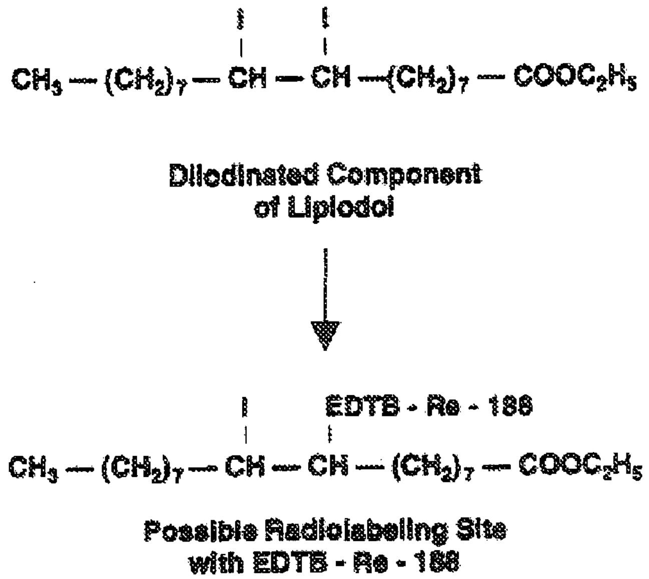


Cependant, cette technique s'est avérée peu pratique et non reproductible. La deuxième stratégie consiste en la solubilisation dans le Lipiodol d'un complexe chimiquement neutre et lipophile de rhénium et est actuellement la voie la plus développée. Des essais cliniques de phase I sont en cours avec le $^{188}$ Re-HDD/Lipiodol (B. Lambert et al., J. Nucl. Med., 2005, 46, 60-66) (HDD = 4-hexadecyl-2, 2, 9, 9-tetramethyl-4, 7-diaza-1, 10-decanethiol). Malheureusement, les rendements de marquage obtenus sont d'environ 50 %, ce qui est un obstacle à l'obtention d'activités importantes (B. Lambert et al., Eur. J. Nucl. Med., 2006, 33, 344-352). D'autres complexes que le Re-HDD ont ainsi été proposés, comme le ReN-DEDC (bis-(diethyldithiocarbamato)nitrido rhenium) (A. Boschi et al., Nucl. Med. Commun., 2004, 25, 691-699), qui, lui, présente un problème de stabilité à 24 h, dû à la radiolyse du complexe à haute activité. Ces problèmes de stabilité des formulations dans l'art antérieur altèrent l'efficacité des compositions et rend très difficile leur exploitation pour une utilisation en clinique. Un kit de marquage, basé sur le complexe SSS (« Super Six Sulphur », bis(trithioperoxybenzoate)(dithiobenzoate) rhénium (III)) décrit par F. Mévellec *et al.* (F. Mévellec et al., Inorg. Chem. Comm., 1999, 2, 230-233 ; WO 01/90114 Al), a montré de bons résultats à basse activité. Cependant, cette demande de brevet s'intéresse principalement au marquage des lymphocytes par du $^{99}Tc$ et utilise des plages de radioactivité relativement basses (0,4-0,8 Gbq). Une autre étude a mené à la conception d'un kit pour la synthèse de $^{188}Re$ (III). Ces complexes, au degré d'oxydation III, sont particulièrement intéressants dans des produits radiopharmaceutiques car ils présentent une meilleure stabilité chimique et thermodynamique que les produits radiopharmaceutiques dans lesquels le métal radioactif est au degré d'oxydation V, comme c'est le cas pour le HDD et le DEDC. La composition obtenue au moyen de ce kit, comprenant le complexe $^{188}Re$-SSS à basse activité et le Lipiodol, est stable au moins 48h et fixe préférentiellement la tumeur hépatique. Brièvement, la méthode de préparation selon ce kit consiste à mélanger en une étape du perrhénate de sodium dans 0,5 ml de solution saline, à 0,8 mg de $SnCl_2$, 7,5 mg de gluconate de sodium, 30 mg d'acide ascorbique et 40 mg d'oxalate de potassium en solution dans 0,5 ml d'une solution saline à 0,9%. Le volume final est donc de 1 ml. La réduction du perrhénate s'opère à température ambiante pendant 15 min pour former un composé intermédiaire. À ce composé intermédiaire sont ajoutés 20 mg de ligand, du dithiobenzoate de sodium. Le mélange doit être chauffé 30 min à 100°C pour obtenir le complexe $[^{188}Re\text{-}(C_6H_5\text{-}CS_3)_2(C_6H_5\text{-}CS_2)]$, extrêmement lipophile. Ce complexe est solubilisé dans 2 à 3 ml de Lipiodol non radioactif. La séparation entre la phase aqueuse contenant l'excès de ligand et la phase lipophile radiomarquée se fait par 10 minutes de centrifugation à 3500 rpm. Ce kit permet la préparation de petites quantités de Lipiodol radiomarqué dont la pureté radiochimique moyenne avoisine

91%. Néanmoins, il ne permet pas le marquage à haute activité du rhénium, les niveaux obtenus étant de l'ordre de 37-370 MBq. De plus, les rendements de marquage et les valeurs de pureté radiochimique sont insuffisants pour la préparation de compositions thérapeutiques utilisant le $^{188}Re$ (N. Lepareur et al., J. Label. Compd. Radiopharm., 2004, 47, 857-867 ; E. Garin et al., Eur. J. Nucl. Med. Mol. Imaging, 2004, 31, 542-546).

Garin et al (Nuclear Medicine Communications 2004, 25:1007-1013) décrivent la préparation d'une composition de $^{188}Re$-SSS lipidiol. L'activité des compositions obtenues est comprise entre 111 et 148 MBq. La stabilité de compositions ayant une activité supérieure à 3,7 GBq est en revanche inconnue et ces compositions ne sont pas préparées dans ce document.

En outre, la préparation de radiotraceurs, et en particulier le marquage du Lipiodol à haute activité de rhénium, entraîne une irradiation non négligeable du personnel manipulant. Dans ce contexte, plusieurs systèmes automatisés ont été décrits dans la littérature, principalement pour la préparation de radiotraceurs fluorés et carbonés, pour l'imagerie par Tomographie par Emission de Positons (TEP), mais également pour la préparation de traceurs rhéniés dont l'activité est supérieure à 15 GBq (S.J. Oh et al., Appl. Radiat. Isot., 2001, 54, 419-427 ; S.J. Oh et al., Appl. Radiat. Isot., 2003, 59, 225-230). Cependant, il n'existe, à notre connaissance, aucun procédé automatisé permettant de préparer des complexes $^{188}Re$-SSS pour la préparation de substance chimiothérapeutiques.

## 4. Objectifs de l'invention

L'invention a notamment pour objectif de pallier ces inconvénients de l'art antérieur.

Plus précisément, un objectif de l'invention est de fournir une composition pour le traitement du cancer du foie chez l'homme qui soit stable, efficace et dont la préparation est sûre pour le manipulateur.

Un autre objectif de l'invention est de mettre en oeuvre un procédé automatisé de préparation d'une telle composition.

## 5. Exposé de l'invention

Ces objectifs, ainsi que d'autres qui apparaîtront par la suite sont atteints à l'aide d'une composition pour le traitement du cancer du foie chez l'homme comprenant un complexe de rhénium radioisotopique (III) à haute activité et une phase organique lipophile. On entend par « haute activité radioisotopique » une radioactivité supérieure à 3,7 GBq. Selon l'invention, une telle composition pour le traitement du cancer du foie chez l'homme comprend un complexe de rhénium radioisotopique (III) à haute activité, c'est-à-dire plus de 3,7 GBq, et une phase organique lipophile mise en émulsion avec une phase aqueuse.

L'invention concerne plus particulièrement une composition pour le traitement du cancer du foie chez l'homme comprenant un complexe de formule (I) suivante :

$$[M(RCS_3)_2(RCS_2)] \qquad (I)$$

dans laquelle M est le $^{188}Re$ à haute activité, c'est-à-dire supérieure à 3,7 GBq, et une phase organique lipophile mise en émulsion avec une phase aqueuse, R représentant un groupe alkyle, cycloalkyle, aralkyle ou aryle, non substitué ou substitué par un ou plusieurs substituants choisis parmi les atomes d'halogène, le groupe hydroxyle, les groupes alkyle et les groupes alcoxy.

Les groupes alkyles utilisés pour R peuvent être des groupes linéaires ou ramifiés en $C_1$ à $C_{12}$, de préférence des groupes ayant 3 à 12 atomes de carbone. Les groupes cycloalkyles utilisés pour R ont de préférence 3 à 7 atomes de carbone, par exemple 6 atomes de carbone. Les groupes aryles utilisés pour R peuvent être du type phényle ou naphtyle. Les groupes aralkyles utilisés pour R peuvent être du type $C_6H_5(CH_2)_n$ avec n allant de 1 à 3, de préférence n est égal à 1 ou 2.

De préférence, selon l'invention, le groupe R est un groupe aryle, aralkyle ou cyclohexyle, éventuellement substitué.

Avantageusement, lorsque R est un groupe aryle, il est choisi parmi les groupes phényle, phényle substitué par un ou plusieurs groupes méthyle, éthyle, propyle, butyle, éthoxy, méthoxy et/ou hydroxyle, et/ou par un ou plusieurs atomes de fluor, chlore, brome et/ou iode, naphtyle et naphtyle substitué par un groupe choisi parmi les groupes alkyle, alcoxy et les atomes d'halogène.

Dans le cas où R est un groupe aralkyle, celui-ci est avantageusement le groupe benzyle ou phénéthyle.

La composition comprend également une phase organique lipophile, qui peut-être un solvant organique ou une huile. Dans le cas où la phase organique est une huile, elle sera préférentiellement un mélange d'esters d'acides gras iodés.

De préférence, cette huile sera du Lipiodol, un mélange d'esters méthyliques iodés de l'huile d'oeillette, huile produite à base de graine de pavot.

L'utilisation du Lipiodol dans une composition selon la présente invention permet d'obtenir une composition stable. De plus, elle est caractérisée par une fixation tumorale préférentielle et durable, permettant ainsi de traiter efficacement le cancer du foie.

Avantageusement, la composition selon la présente invention comprend une phase organique lipophile qui peut-être mise en émulsion avec une phase aqueuse. De préférence, la phase aqueuse est du sérum physiologique. Ceci permet d'améliorer sa biodistribution.

La composition pour le traitement du cancer du foie comprenant un complexe de rhénium radioisotopique de formule (I) à haute activité et la phase organique lipophile peut être préparée en moins d'une heure à partir d'un kit comportant trois récipients contenant respectivement :

- le réducteur et des additifs (sel d'étain, gluconate, oxalate, antioxydant),-le dithiocarboxylate ($RCS_2^-$ $Z1^+$) dans lequel R est tel que défini ci-dessus et Z1 représente un cation pharmaceutiquement acceptable, et
- une phase organique lipophile.

Les cations pharmaceutiquement acceptables utilisés pour Z1 peuvent être choisis parmi les cations de formule générale $MgX^+$ où X est un atome d'halogène tel que Br ou Cl, les cations ammonium quaternaire, ainsi que les ions de métal alcalin tel que Na.

La composition pour le traitement du cancer du foie comprenant un complexe de rhénium radioisotopique de formule (I) à haute activité et la phase organique lipophile peut être préparée à l'aide d'un système automatisé, et notamment l'automate TADDEO® de Comecer (Italie).

Le procédé automatisé de l'invention comprend les étapes suivantes :

- synthèse du complexe de formule (I),
- purification du complexe de formule (I) sur colonne
- évaporation du solvant organique, préférentiellement à une température comprise entre 40°C et 100°C.
- filtration stérilisante
- solubilisation du complexe de formule (I) dans la phase organique lipophile, de préférence le Lipiodol.

Le complexe de formule (I) est synthétisé en deux étapes.

La première étape consiste en une réduction de l'ion perrhénate de formule $[^{188}Re^{VII}O_4]^-$, dans un volume de 0,5ml à 2 ml, en présence de :

- 2,6 à 4 mg/ml de chlorure d'étain,
- 20 à 30 mg/ml de gluconate de sodium,
- 26 à 40 mg/ml d'oxalate de potassium et
- 20 à 30 mg/ml d'acide ascorbique.

On obtient ainsi un composé neutre radioactif dont le degré d'oxydation du $^{188}Re$ est abaissé, par exemple de VII à V. Il est toutefois utile d'utiliser un antioxydant en parallèle de la réduction pour éviter que le complexe intermédiaire ne s'oxyde à nouveau en ion perrhénate. La période d'incubation à température ambiante pour que la réaction de réduction s'opère est ramenée entre 5 à 15 minutes.

La seconde étape consiste à faire réagir ce composé un ion de formule générale $R\text{-}CS_2^-$, dans lequel R représente un groupe alkyle, cycloalkyle, aralkyle ou aryle, non substitué ou substitué par un ou plusieurs substituants choisis parmi les atomes d'halogène, le groupe hydroxyle, les groupes alkyle et les groupes alcoxy. La réaction entre le composé intermédiaire et le ligand s'opère par 15 min de chauffage à 100°C. On obtient ainsi en deux étapes le composé final de formule $[^{188}Re\ (RCS_3)_2(RCS_2)]$. Ce complexe est extrêmement lipophile.

Il est important que le volume de réaction de synthèse du composé (I) soit faible, de l'ordre de 0,5 à 2 ml. L'utilisation d'un volume plus élevé a un effet négatif sur la qualité de la formation du complexe et la pureté radiochimique.

La purification s'effectue par chromatographie, notamment en Chromatographie Liquide à Haute Performance (HPLC). La purification sur colonne peut se faire sur une colonne C8 ou encore sur une colonne C18. On entend par colonne C8 ou C18 des colonnes pour HPLC en phase inverse dont la phase stationnaire est une phase apolaire constituée de gel de silice sur lequel sont greffées des chaînes de 8 ou 18 atomes de Carbone. En ce cas, l'élution se fait en utilisant une phase mobile polaire et se choisit en fonction de la nature chimique des molécules à éluer.

Après élution, le complexe purifié subit une filtration stérilisante avant d'être solubilisé dans 2 à 3 ml de Lipiodol non radioactif. On entend par filtration stérilisante le passage du complexe en solution à travers un filtre de porosité 0,2μm pour éliminer les micro-organismes pathogènes.

L'automatisation du procédé rend possible une utilisation de cette formulation à des fins cliniques et pharmaceutiques en limitant grandement l'exposition du manipulateur à la radioactivité. Ainsi, le procédé selon la présente

invention permet d'obtenir, par rapport à l'art antérieur, des compositions stables et efficaces, compatibles avec une utilisation thérapeutique concrète. De plus, le temps de préparation est considérablement diminué et la reproductibilité est largement améliorée en comparaison du kit développé dans la publication de N. Lepareur et al. (J. Label. Compd. Radiopharm., 2004, 47, 857-867).

**7. Description de différents modes de réalisation de l'invention**

Les modes de réalisation suivant sont donnés à titre d'exemples de la présente invention.

La figure 1 représente le schéma du procédé automatisé.

**Exemple 1 : Préparation d'un complexe [$^{188}Re$-($C_6H_5$-$CS_3$)$_2$($C_6H_5$-$CS_2$)] ($^{188}Re$-SSS) à haute activité**

On mélange dans un récipient adapté 4 mg de chlorure d'étain, 30 mg de gluconate de sodium, 60 mg d'oxalate de potassium et 30 mg d'acide ascorbique. L'ensemble de ces substances lyophilisées est reconstitué dans 0,5 mL de sérum physiologique. La solution de $^{188}Re$-perrhénate (11,1 GBq, 1 mL) est ensuite ajoutée. Le mélange ainsi obtenu est agité 15 minutes à température ambiante. À ce mélange sont ajoutés 40 mg de ligand ($C_6H_5$)-$CS_2Na$ (0,2 mmoles), en solution dans 0,5 mL de sérum physiologique. Le mélange est chauffé 15 minutes à 100°C pour donner le complexe [$^{188}Re$-($C_6H_5$-$CS_3$)$_2$($C_6H_5$-$CS_2$)].

**Exemple 2: Préparation d'un composé marqué au $^{188}Re$ (III) à haute activité en solution dans le Lipiodol**

Au mélange réactionnel obtenu dans l'exemple 1, on ajoute 2 mL de Lipiodol. Le mélange est agité 10 minutes puis centrifugé à 3500 tours/min pendant 10 minutes supplémentaires. La phase aqueuse contenant les réactifs en excès est éliminée et la phase Lipiodolée radiomarquée est recueillie.
Rendement de marquage = 98,6 %
Pureté radiochimique (PRC) = 96 %
Chromatographie sur Couche Mince sur gel de silice (notée CCM pour la suite): $R_f$ = 0,62

Le rendement de marquage en pourcentage est calculé de la façon suivante :

$$\text{Rendement de marquage (\%)} = [\text{activité de la phase lipophile (Bq)} \times 100] / [\text{activité de la phase lipophile (Bq)} + \text{activité de la phase aqueuse (Bq)}]$$

La pureté radiochimique compatible avec une application pharmaceutique se définit comme au moins 95% de complexe $^{188}Re$ (III) contenus dans la phase lipophile.

L'analyse de chromatographie sur couche mince sur plaque de gel de silice a pour objectif de déterminer la pureté radiochimique de la solution préparée. L'éluant utilisé pour effectuer la migration est un mélange composé d'éther de pétrole et de dichlorométhane dans des proportions volumiques respectives de 60%/40% (v/v). La chromatographie est ensuite effectuée selon les méthodes bien connues de l'homme de l'art. Brièvement, une goutte de la solution radiomarquée est déposée sur une bande CCM, placée dans une chambre de développement. Un solvant, ici le mélange d'éther de pétrole et de dichlorométhane, est disposé dans le réservoir de la chambre sans toucher la bande. La chambre est refermée et la migration du front de solvant s'opère, entraînant avec lui l'échantillon de solution radiomarquée. A la fin de la migration, la bande est coupée en deux moitiés et l'activité de chaque bande est mesurée avec un compteur Béta ou tout autre compteur adapté à la mesure de la radioactivité. Les plaques chromatographiques sont visualisées, par exemple à l'aide du phospho-imageur Fujix BAS 1000.
La Pureté Radiochimique (notée PRC) est exprimée en % et calculée de la façon suivante :

$$\text{PRC} = [\text{activité du spot radioactif d'intérêt (Bq)} \times 100] / [\text{activité totale (Bq)}]$$

Le ratio de migration de la radioactivité $R_f$ = (radioactivité du front de migration du solvant entraînant le composé (Bq)) / (radioactivité totale de la bande CCM (Bq))

**Exemple 3: Préparation d'un composé marqué au $^{188}Re$ (III) à haute activité en solution dans le Lipiodol**

Au mélange réactionnel obtenu comme dans l'exemple 1, avec le ligand 4-$CH_3$-O-($C_6H_4$)-$CS_2Na$ (47,6 mg,

0,23 mmoles), on ajoute 2 mL de Lipiodol. Le mélange est agité 10 min puis centrifugé à 3500 tours/min pendant 10 min supplémentaires. La phase aqueuse est éliminée et la phase Lipiodolée radiomarquée est recueillie.
Rendement de marquage= 98 %
Pureté radiochimique= 97 %
CCM: $R_f$= 0,25

**Exemple 4: Préparation d'un composé marqué au $^{188}$Re (III) à haute activité en solution dans le Lipiodol**

Au mélange réactionnel obtenu comme dans l'exemple 1, avec le ligand $CH_3(CH_2)_{11}CS_2Na$ (61 mg, 0,2 mmoles), on ajoute 2 mL de Lipiodol. Le mélange est agité 10 min puis centrifugé à 3500 tours/min pendant 10 min supplémentaires. La phase aqueuse est éliminée et la phase Lipiodolée radiomarquée est recueillie.
Rendement de marquage= 32 %
Pureté radiochimique = 97 %
CCM : $R_f$= 0,90

**Exemple 5: Préparation automatisée (TADDEO®) d'un composé marqué au $^{188}$Re (III) à haute activité en solution dans le Lipiodol**

En référence à la figure 1, on place dans le récipient A 4 mg de chlorure d'étain, 30 mg de gluconate de sodium, 40 mg d'oxalate de potassium et 30 mg d'acide ascorbique. Ce mélange de substances lyophilisées est reconstitué par 1 mL de $^{188}$Re-perrhénate (8 GBq). Après 5 min d'incubation à température ambiante, la solution est automatiquement transférée dans le réacteur R par flux d'air à 1,5 bars. Un flacon B, contenant 20 mg de ligand $(C_6H_5)$-$CS_2Na$ (0,2 mmoles), est reconstitué par vidange de la fiole F2 contenant 0,5 mL d'éthanol + 0,3 mL de Lipiodol. La solution est transférée de B vers R par flux d'air à 1,5 bar. Le réacteur R est chauffé 12 minutes à 100°C pour donner le complexe [$^{188}$Re-$(C_6H_5$-$CS_3)_2(C_6H_5$-$CS_2)$], puis refroidi par souffle d'air à 2 bars sur le réacteur pendant 7 minutes. Le milieu réactionnel est ensuite transféré sur une colonne de HPLC en phase inverse Sep-Pak® C18 et lavé par 20 mL d'eau distillée (F3) puis par 2 mL d'un mélange eau distillée/éthanol 1/1 (F4). Le complexe [$^{188}$Re-$(C_6H_5$-$CS_3)_2(C_6H_5$-$CS_2)$] est ensuite élué dans le flacon C par 5 mL d'éthanol (F5). L'éthanol est évaporé à 85 °C et 2 mL de Lipiodol (F6) sont ajoutés pour donner la composition radiomarquée désirée.
Rendement de marquage = 60 %
Pureté radiochimique= 91,2 %
CCM: $R_f$= 0,62

**Exemple 6: Préparation automatisée (TADDEO®) d'un composé marqué au $^{188}$Re (III) à haute activité (> 3,7 GBq) en solution dans le Lipiodol**

En référence, à la figure 1, le récipient A contient 4 mg de chlorure d'étain, 30 mg de gluconate de sodium, 40 mg d'oxalate de potassium et 30 mg d'acide ascorbique. Ce mélange de substances lyophilisées est reconstitué par 1,5 mL de $^{188}$Re-perrhénate (7,65 GBq). Après 5 min à température ambiante, la solution est automatiquement transférée dans le réacteur R par flux d'air à 1,5 bars. Le flacon B, contenant 40 mg de ligand $(C_6H_5)$-$CS_2Na$ (0,4 mmoles), est reconstitué par vidange de la fiole F2 (1 mL d'éthanol + 0,3 mL de Lipiodol). La solution est transférée de B vers R par flux d'air à 1,5 bars. Le réacteur R est chauffé 15 minutes à 100°C pour donner le complexe [$^{188}$Re-$(C_6H_5$-$CS_3)_2$ $(C_6H_5$-$CS_2)$], puis refroidi par souffle d'air à 2 bars sur le réacteur pendant 7 minutes. Le milieu réactionnel est ensuite transféré sur une colonne Sep-Pak® C18 et lavé par 10 mL d'eau distillée (F3) puis par 2 mL d'un mélange eau distillée/ éthanol 1/1 (F4). Le complexe [$^{188}$Re-$(C_6H_5$-$CS_3)_2(C_6H_5$-$CS_2)$] est ensuite élué dans le flacon C par 5 mL d'éthanol (F5). L'éthanol est évaporé à 100 °C et 2 mL de Lipiodol (F6) sont ajoutés pour donner la composition radiomarquée désirée.
Rendement de marquage= 59,5 % -
Pureté radiochimique= 94 %
CCM: $R_f$= 0,62

**Exemple 7: Préparation automatisée (TADDEO®) d'un composé marqué au $^{188}$Re (III) en solution dans le Lipiodol**

En référence à la figure 1, le récipient A contient 4 mg de chlorure d'étain, 30 mg de gluconate de sodium, 40 mg d'oxalate de potassium et 30 mg d'acide ascorbique. Ce mélange de substances lyophilisées est reconstitué par 1 mL de $^{188}$Re-perrhénate (0,2 GBq). Après 5 min à température ambiante, la solution est automatiquement transférée dans le réacteur R par flux d'air à 1,5 bars. Le flacon B, contenant 40 mg de ligand $(C_6H_5)$-$CS_2Na$ (0,4 mmoles), est reconstitué par vidange de la fiole F2 (1 mL d'éthanol + 0,5 mL de Lipiodol). La solution est transférée de B vers R par

flux d'air à 1,5 bars. Le réacteur R est chauffé 15 minutes à 100°C pour donner le complexe [$^{188}Re-(C_6H_5-CS_3)_2(C_6H_5-CS_2)$], puis refroidi par souffle d'air à 2 bars sur le réacteur pendant 7 minutes. Le milieu réactionnel est ensuite transféré sur une colonne Sep-Pak® C8 et lavé par 10 mL d'eau distillée (F3) puis par 2 mL d'un mélange eau distillée/éthanol 1/1 (F4). Le complexe [$^{188}Re-(C_6H_5-CS_3)_2(C_6H_5-CS_2)$] est ensuite élué dans le flacon C par 2,5 mL d'éthanol (F5). L'éthanol est évaporé à 100°C et 2 mL de Lipiodol (F6) sont ajoutés pour donner la composition radiomarquée désirée.
Rendement de marquage= 67,4 %
Pureté radiochimique= 90,1 %
CCM: $R_f$ = 0,62

**Exemple 8: Stabilité du composé marqué au $^{188}$Re (III) à haute activité en solution dans le Lipiodol**

La composition radiomarquée obtenue selon l'exemple 2 est mise à incuber en présence de sérum physiologique pendant 64 h. Puis on procède à une séparation des phases comme précédemment. Le mélange est agité 10 minutes puis centrifugé à 3500 tours/min pendant 10 minutes supplémentaires. La phase aqueuse est éliminée et la phase lipiodolée radiomarquée est recueillie. La radioactivité de chaque phase, aqueuse et lipophile, est comptée pour vérifier qu'il n'y a pas de redistribution de la radioactivité dans la phase aqueuse (radiolyse du complexe). 97% de la radioactivité reste dans le Lipiodol, et la pureté radiochimique du complexe est restée inchangée.

**Exemple 9: Stabilité du composé marqué au $^{188}$Re (III) à haute activité en solution dans le Lipiodol**

Une autre composition radiomarquée obtenue selon l'exemple 2 (radioactivité = 10,3 GBq ; rendement de marquage= 97,8% ; pureté radiochimique = 92,1 %) est mise à incuber en présence de sérum physiologique pendant 168 h. Puis on procède à une séparation des phases comme précédemment. Le mélange est agité 10 minutes puis centrifugé à 3500 tours/min pendant 10 minutes supplémentaires. La phase aqueuse est éliminée et la phase lipiodolée radiomarquée et recueillie. Les deux phases sont comptées pour vérifier qu'on n'a pas de redistribution de la radioactivité dans la phase aqueuse (radiolyse du complexe). 99,6 % de la radioactivité reste dans le Lipiodol, et la pureté radiochimique du complexe est restée similaire (pureté radiochimique= 90,2 %). Un lavage au sérum physiologique, comme il est décrit dans l'art antérieur (B. Lambert et al., J.Nucl. Med., 2005, 46, 60-66), n'est ici pas nécessaire, le complexe étant produit avec une pureté radiochimique suffisante.

Ce procédé permet d'obtenir un composé stable tout en simplifiant le mode de préparation puisqu'il se fait principalement en phase aqueuse. Ce procédé est par conséquent tout à fait adapté à la préparation de compositions chimiothérapeutiques à haute activité.

## Revendications

1. Composition pour une utilisation dans le traitement du cancer du foie chez l'homme comprenant un complexe de formule (I) suivante :

$$[M(RCS_3)_2(RCS_2)] \qquad (I)$$

dans laquelle M est le $^{188}$Re à haute activité, c'est-à-dire supérieure à 3,7 GBq, et une phase organique lipophile mise en émulsion avec une phase aqueuse, R représentant un groupe alkyle, cycloalkyle, aralkyle ou aryle, non substitué ou substitué par un ou plusieurs substituants choisis parmi les atomes d'halogène, le groupe hydroxyle, les groupes alkyle et les groupes alcoxy.

2. Composition pour une utilisation selon la revendication 1, dans lequel R est le groupe phényle.

3. Composition pour une utilisation selon la revendication 1, dans lequel R est un groupe alkyle, linéaire ou ramifié, en $C_1$ à $C_{12}$.

4. Composition pour une utilisation selon la revendication 1, dans lequel R représente le groupe naphtyle ou le groupe naphtyle substitué par un groupe choisi parmi les groupes alkyle, alcoxy et les atomes d'halogène.

5. Composition pour une utilisation selon la revendication 1, dans lequel R est choisi parmi les groupes phényle substitué par un ou plusieurs groupes méthyle, éthyle, propyle, butyle, méthoxy, éthoxy et/ou hydroxyle, et/ou par un ou plusieurs atomes de fluor, chlore, brome et/ou iode.

**6.** Composition pour une utilisation selon la revendication 1, dans lequel R est le groupe cyclohexyle, benzyle ou phénéthyle.

**7.** Composition pour une utilisation selon les revendications de 1 à 6, dans laquelle la phase organique lipophile est une huile.

**8.** Composition pour une utilisation selon la revendication 7, dans laquelle l'huile est un mélange d'esters d'acides gras iodés.

**9.** Composition pour une utilisation selon la revendication 8, dans laquelle l'huile est du Lipiodol.

**10.** Composition pour une utilisation selon les revendications 1 à 6, dans laquelle la phase organique lipophile est un solvant organique.

**11.** Composition pour une utilisation selon la revendication 10, dans laquelle la phase aqueuse est du sérum physiologique.

**12.** Procédé de préparation d'une composition selon l'une quelconque des revendications de 1 à 11, **caractérisé en ce qu'**il inclut les étapes suivantes :

a) synthèse du complexe de formule (I) en 2 étapes dans un volume final de 0,5 à 2 ml d'une solution aqueuse:

- la première étape consistant en une réduction de l'ion perrhénate $^{188}ReO_4^-$ en présence de 2,6 à 4 mg/ml de chlorure d'étain, de 20 à 30 mg/ml de gluconate de sodium, de 26 à 40 mg/ml d'oxalate de potassium et de 20 à 30 mg/ml d'acide ascorbique pour former un composé intermédiaire; et
- la seconde étape consistant en la réaction du composé intermédiaire avec un ion de formule générale $R-CS_2^-$, pour former le complexe de formule (I), R représentant un groupe alkyle, cycloalkyle, aralkyle ou aryle, non substitué ou substitué par un ou plusieurs substituants choisis parmi les atomes d'halogène, le groupe hydroxyle, les groupes alkyle et les groupes alcoxy ;

b) purification du complexe de formule (I) sur colonne ;
c) évaporation du solvant organique ;
d) filtration stérilisante ;
e) solubilisation du complexe de formule (I) dans la phase organique,
f) mise en émulsion avec une phase aqueuse.

**13.** Procédé selon la revendication 12, dans lequel le complexe de formule (I) est purifié sur une colonne de chromatographie liquide haute performance, dite HPLC.

**14.** Procédé selon la revendication 13, dans lequel la colonne est une C8.

**15.** Procédé selon la revendication 13, dans lequel la colonne est une C18.

**16.** Procédé selon la revendication 12, dans lequel le solvant organique est évaporé à une température comprise entre 40°C et 100°C.

## Patentansprüche

**1.** Zusammensetzung zur Anwendung bei der Behandlung von Leberkrebs beim Menschen, umfassend einen Komplex mit der folgenden Formel (I):

$$[M(RCS_3)_2(RCS_2)] \qquad (I)$$

wobei M das $^{188}Re$ mit hoher Aktivität, das heißt größer als 3,7 GBq, ist und eine organische lipophile Phase, die mit einer wässrigen Phase emulgiert ist, wobei R für eine Alkyl-, Cycloalkyl-, Aralkyl- oder Arylgruppe steht, die mit einem oder mehreren Substituenten substituiert oder nicht substituiert ist, ausgewählt aus den Halogenatomen, der Hydroxylgruppe, den Alkylgruppen und den Alkoxygruppen.

2. Zusammensetzung zur Anwendung nach Anspruch 1, wobei R die Phenylgruppe ist.

3. Zusammensetzung zur Anwendung nach Anspruch 1, wobei R eine lineare oder verzweigte $C_1$- bis $C_{12}$-Alkylgruppe ist.

4. Zusammensetzung zur Anwendung nach Anspruch 1, wobei R für die Naphtylgruppe oder die mit einer Gruppe, ausgewählt aus den Alkyl-, Alkoxygruppen und den Halogenatomen, substituierte Naphtylgruppe steht.

5. Zusammensetzung zur Anwendung nach Anspruch 1, wobei R ausgewählt ist aus den Phenylgruppen, die mit einer oder mehreren Methyl-, Ethyl-, Propyl-, Butyl-, Methoxy-, Ethoxy- und/oder Hydroxylgruppen, und/oder mit einem oder mehreren Fluor-, Chlor-, Brom- und/oder Iodatomen substituiert ist.

6. Zusammensetzung zur Anwendung nach Anspruch 1, wobei R die Cyclohexyl-, Benzyl- oder Phenethylgruppe ist.

7. Zusammensetzung zur Anwendung nach den Ansprüchen 1 bis 6, wobei die organische lipophile Phase ein Öl ist.

8. Zusammensetzung zur Anwendung nach Anspruch 7, wobei das Öl ein Gemisch aus iodierten Fettsäureestern ist.

9. Zusammensetzung zur Anwendung nach Anspruch 8, wobei das Öl Lipiodol ist.

10. Zusammensetzung zur Anwendung nach den Ansprüchen 1 bis 6, wobei die organische lipophile Phase ein organisches Lösemittel ist.

11. Zusammensetzung zur Anwendung nach Anspruch 10, wobei die wässrige Phase physiologische Kochsalzlösung ist.

12. Verfahren zur Herstellung einer Zusammensetzung nach einem der Ansprüche 1 bis 11, **dadurch gekennzeichnet, dass** es die folgenden Schritte einschließt:

a) Synthetisieren des Komplexes mit der Formel (I) in 2 Schritten in einem Endvolumen von 0,5 bis 2 ml einer wässrigen Lösung:

- wobei der erste Schritt in einer Reduktion des Perrhenations $^{188}ReO_4^-$ in Gegenwart von 2,6 bis 4 mg/ml Zinnchlorid, 20 bis 30 mg/ml Natriumgluconat, 26 bis 40 mg/ml Kaliumoxalat und 20 bis 30 mg/ml Ascorbinsäure besteht, um eine Zwischenverbindung zu bilden; und
- der zweite Schritt in der Reaktion der Zwischenverbindung mit einem Ion der allgemeinen Formel $R\text{-}CS_2^-$ besteht, um den Komplex mit der Formel (I) zu bilden, wobei R für eine Alkyl-, Cycloalkyl-, Aralkyl- oder Arylgruppe steht, die mit einem oder mehreren Substituenten substituiert oder nicht substituiert ist, ausgewählt aus den Halogenatomen, der Hydroxylgruppe, den Alkylgruppen und den Alkoxygruppen.

b) Reinigen des Komplexes mit der Formel (I) auf der Säule;
c) Verdampfen des organischen Lösemittels;
d) Sterilfiltrieren;
e) Solubilisieren des Komplexes mit der Formel (I) in der organischen Phase,
f) Emulgieren mit einer wässrigen Phase.

13. Verfahren nach Anspruch 12, wobei der Komplex mit der Formel (I) auf einer Hochleistungsflüssigchromatographiesäule, genannt HPLC, gereinigt wird.

14. Verfahren nach Anspruch 13, wobei die Säule eine C8-Säule ist.

15. Verfahren nach Anspruch 13, wobei die Säule eine C18-Säule ist.

16. Verfahren nach Anspruch 12, wobei das organische Lösemittel bei einer Temperatur im Bereich zwischen 40 °C und 100 °C verdampft wird.

## Claims

1. Composition for use in the treatment of liver cancer in humans, comprising a complex of the following formula (I):

$$[M(RCS_3)_2(RCS_2)] \qquad (I)$$

in which M is $^{188}Re$ having high activity, i.e. greater than 3.7 GBq, and an organic lipophilic phase emulsified with an aqueous phase, R representing an alkyl, cycloalkyl, aralkyl or aryl group, unsubstituted or substituted by one or more substituents chosen from halogen atoms, a hydroxyl group, alkyl groups and alkoxy groups.

2. Composition for use according to claim 1, wherein R is a phenyl group.

3. Composition for use according to claim 1, wherein R is a linear or branched $C_1$-to $C_{12}$-alkyl group.

4. Composition for use according to claim 1, wherein R represents a naphthyl group or a naphthyl group substituted by a group chosen from alkyl groups, alkoxy groups and halogen atoms.

5. Composition for use according to claim 1, wherein R is chosen from phenyl groups substituted by one or more methyl, ethyl, propyl, butyl, methoxy, ethoxy and/or hydroxy groups and/or by one or more fluorine, chlorine, bromine and/or iodine atoms.

6. Composition for use according to claim 1, wherein R is a cyclohexyl, benzyl or phenethyl group.

7. Composition for use according to claims 1 to 6, wherein the organic lipophilic phase is an oil.

8. Composition for use according to claim 7, wherein the oil is a mixture of iodinated fatty acid esters.

9. Composition for use according to claim 8, wherein the oil is Lipiodol.

10. Composition for use according to claims 1 to 6, wherein the organic lipophilic phase is an organic solvent.

11. Composition for use according to claim 10, wherein the aqueous phase is physiological serum.

12. Process for the preparation of a composition according to any one of claims 1 to 11, **characterised in that** it includes the following steps:

    a) synthesis of the complex of formula (I) in two steps in a final volume of from 0.5 to 2 ml of an aqueous solution:

        - the first step consisting in reducing the perrhenate ion $^{188}ReO_4^-$ in the presence of from 2.6 to 4 mg/ml of tin chloride, from 20 to 30 mg/ml of sodium gluconate, from 26 to 40 mg/ml of potassium oxalate and from 20 to 30 mg/ml of ascorbic acid to form an intermediate compound; and
        - the second step consisting in reacting the intermediate compound with an ion of the general formula R-$CS_2^-$ to form the complex of formula (I), R representing an alkyl, cycloalkyl, aralkyl or aryl group, unsubstituted or substituted by one or more substituents chosen from halogen atoms, a hydroxyl group, alkyl groups and alkoxy groups;

    b) purification of the complex of formula (I) on a column;
    c) evaporation of the organic solvent;
    d) sterilising filtration;
    e) solubilisation of the complex of formula (I) in the organic phase;
    f) emulsification with an aqueous phase.

13. Process according to claim 12, wherein the complex of formula (I) is purified on a high-performance liquid chromatography (HPLC) column.

14. Process according to claim 13, wherein the column is a C8 column.

15. Process according to claim 13, wherein the column is a C18 column.

**16.** Process according to claim 12, wherein the organic solvent is evaporated at a temperature of from 40°C to 100°C.

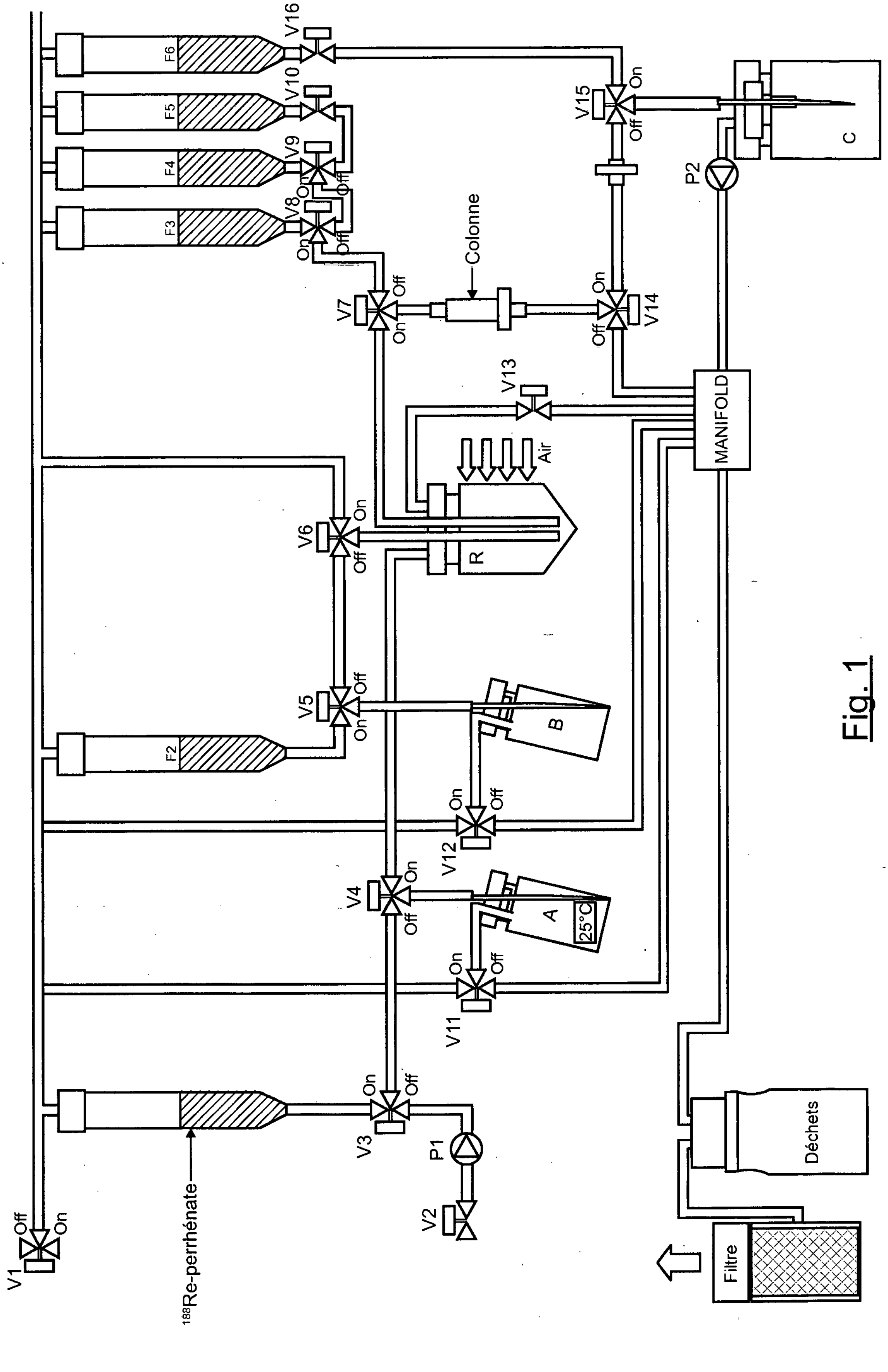
V1
Off
On
188Re-perrhénate
V2
P1
V3
On
Off
V4
V11
V12
V5
V6
V7
V8
V9
V10
V13
V14
V15
V16
F2
F3
F4
F5
F6
A
25°C
B
R
Air
Colonne
MANIFOLD
P2
C
Déchets
Filtre

Fig. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0190114 A1


### Littérature non-brevet citée dans la description


- **E. GARIN ; P. BOURGUET.** Nuclear Medicine in Clinical Diagnosis and Treatment, Hong-Kong. 2004, 473-483
- **B. LAMBERT.** *J.Nucl.Med.,* 2005, vol. 46, 60-66
- **S.J. WANG et al.** *J. Nucl. Med.,* 1996, vol. 37, 332-335
- **S.J. WANG et al.** *Appl. Radiat. Isot.,* 1996, vol. 47, 267-271
- **B. LAMBERT et al.** *J. Nucl. Med.,* 2005, vol. 46, 60-66
- **B. LAMBERT et al.** *Eur. J. Nucl. Med.,* 2006, vol. 33, 344-352
- **A. BOSCHI et al.** *Nucl. Med. Commun.,* 2004, vol. 25, 691-699
- **F. MÉVELLEC et al.** *Inorg. Chem. Comm.,* 1999, vol. 2, 230-233
- **N. LEPAREUR et al.** *J. Label. Compd. Radiopharm.,* 2004, vol. 47, 857-867
- **E. GARIN et al.** *Eur. J. Nucl. Med. Mol. Imaging,* 2004, vol. 31, 542-546
- **GARIN et al.** *Nuclear Medicine Communications,* 2004, vol. 25, 1007-1013
- **S.J. OH et al.** *Appl. Radiat. Isot.,* 2001, vol. 54, 419-427
- **S.J. OH et al.** *Appl. Radiat. Isot.,* 2003, vol. 59, 225-230
- **LEPAREUR et al.** *J. Label. Compd. Radiopharm.,* 2004, vol. 47, 857-867
- **B. LAMBERT et al.** *J.Nucl. Med.,* 2005, vol. 46, 60-66